(19) Europäisches Patentamt
European Patent Office
Office européen des brevets

(11) Veröffentlichungsnummer: **0 029 983**
**A1**

# (12) EUROPÄISCHE PATENTANMELDUNG

(21) Anmeldenummer: 80107307.3

(22) Anmeldetag: 24.11.80

(51) Int. Cl.³: C 07 D 471/04
A 61 K 31/64
//(C07D471/04, 221/00, 209/00)

(30) Priorität: 01.12.79 DE 2948522

(43) Veröffentlichungstag der Anmeldung:
10.06.81 Patentblatt 81/23

(84) Benannte Vertragsstaaten:
AT BE CH DE FR GB IT LI LU NL SE

(71) Anmelder: HOECHST AKTIENGESELLSCHAFT
Zentrale Patentabteilung Postfach 80 03 20
D-6230 Frankfurt/Main 80(DE)

(72) Erfinder: Hitzel, Volker, Dr.
Kantstrasse 1b
D-6238 Hofheim am Taunus(DE)

(72) Erfinder: Weyer, Rudi, Dr.
Johann-Strauss-Strasse 43
D-6233 Kelkheim (Taunus)(DE)

(72) Erfinder: Geisen, Karl, Dr.
Jahnstrasse 43
D-6000 Frankfurt am Main(DE)

(72) Erfinder: Regitz, Günter, Dr.
Dachbergstrasse 68
D-6232 Bad Soden am Taunus(DE)

(54) **Benzolsulfonylharnstoffe, Verfahren zu ihrer Herstellung, pharmazeutische Präparate auf Basis dieser Verbindungen sowie ihre Verwendung.**

(57) Sulfonylharnstoffe der Formel

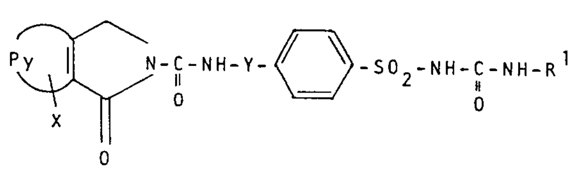

worin 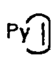, R¹, X und Y die angegebene Bedeutung haben,

sowie deren physiologisch verträgliche Salze, pharmazeutische Präparate auf Basis dieser Verbindungen sowie ihre Verwendung bei der Behandlung des Diabetes.

EP 0 029 983 A1

Croydon Printing Company Ltd.

HOECHST AKTIENGESELLSCHAFT HOE 79/F 329 Dr.D/mh

## Benzolsulfonylharnstoffe, Verfahren zu ihrer Herstellung, pharmazeutische Präparate auf Basis dieser Verbindungen sowie ihre Verwendung

Die Erfindung betrifft Sulfonylharnstoffe der Formel

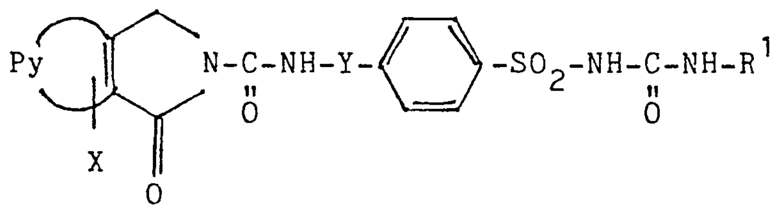

die als Substanz oder in Form ihrer physiologisch verträglichen Salze blutzuckersenkende Eigenschaften besitzen und sich durch starke und langanhaltende Senkung des Blutzuckerspiegels auszeichnen.
In der Formel bedeuten:

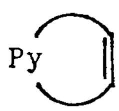 einen Pyridin-Ring, der das Stickstoffatom an den vier möglichen Positionen tragen kann

X Wasserstoff, Alkyl mit 1 - 4 C-Atomen, Halogen

Y Alkylen mit 2 - 3 C-Atomen

$R^1$ Alkyl von 4 - 8 C-Atomen, Cycloalkyl, Alkylcycloalkyl, Dialkylcycloalkyl, Cycloalkylalkyl, Cycloalkenyl, Alkylcycloalkenyl mit jeweils 5 - 9 C-Atomen, Methylcyclopentylmethyl, Cyclohexenylmethyl, Chlorcyclohexyl, Methoxycyclohexyl, Bicycloheptyl, Bicycloheptenyl, Bicyclohetylmethyl, Bicycloheptenylmethyl, Bicyclooctyl, Nortricyclyl, Adamantyl oder Benzyl.

In der allgemeinen Formel bedeutet X vorzugsweise Wasserstoff und Methyl. Besonders bevorzugt ist für X Wasserstoff. Y bedeutet vorzugsweise $-CH_2-CH_2-$, $-\underset{CH_3}{CH}-CH_2-$, wobei die $-CH_2-CH_2$-Gruppe besonders bevorzugt ist. $R^1$ ist vorzugsweise Alkylcyclohexyl, besonders bevorzugt ist 4-Äthylcyclohexyl. Als bicyclische

Reste kommen z.B. in Betracht: Bicyclo/¯2.2.1_7heptyl, Bicyclo/¯2.2.1_7heptyl-methyl sowie die entsprechenden ungesättigten Reste und der Bicyclo/¯2.2.2_7octylrest.

Die Erfindung betrifft ferner Verfahren zur Herstellung dieser Sulfonylharnstoffe, pharmazeutische Präparate, die diese enthalten oder aus ihnen bestehen sowie ihre Verwendung zur Behandlung des Diabetes.

Die Verfahren zur Herstellung sind dadurch gekennzeichnet, daß man

a) mit der Gruppe

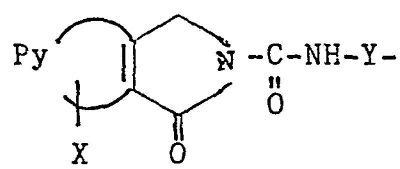

in 4-Stellung substituierte Benzolsulfonyl-isocyanate, -carbaminsäureester, - thiolcarbaminsäureester, -harnstoffe, -semicarbazide oder -semicarbazone mit einem Amin $R^1-NH_2$ oder dessen Salzen umsetzt oder Sulfonamide der Formel

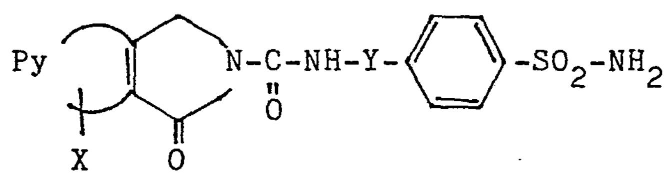

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte Benzolsulfonyl-isoharnstoffäther, -isothioharnstoffäther, -parabansäuren oder -halogenameisensäureamidine spaltet,

c) in

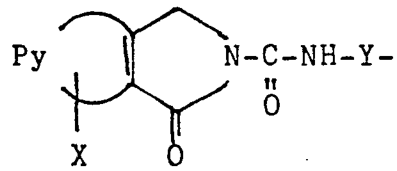

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffen der Formel

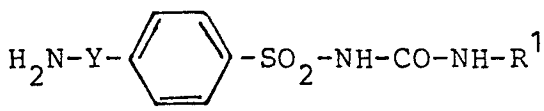

gegebenenfalls stufenweise den Rest

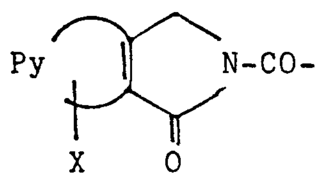

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^1$-N'-hydroxy-harnstoff umsetzt
und die Reaktionsprodukte gegebenenfalls zur Salzbildung mit alkalischen Mitteln behandelt.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der

Reaktion abgespalten wird, hat seine chemische Konstitution keinen Einfluß auf den Charakter des Endproduktes und kann deshalb in weiten Grenzen variiert werden. Das gleiche gilt für die N—$R^1$-substituierten Carbaminsäureester bzw. die entsprechenden Thiolcarbaminsäureester.

Als Carbaminsäurehalogenide eignen sich in erster Linie die Chloride.

Die als Ausgangsstoffe des Verfahrens infrage kommenden Benzolsulfonylharnstoffe können an der der Sulfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen kann man auch Benzolsulfonylcarbamoylimidazole und ähnliche Verbindungen oder Bisbenzolsulfonylharnstoffe, die an einem der Stickstoffatome noch einen weiteren Substituenten, z.B. Methyl, tragen können, verwenden. Man kann beispielsweise derartige Bis-(benzolsulfonyl)-harnstoffe oder auch N-Benzolsulfonyl-N'-acylharnstoffe mit $R^1$-substituierten Aminen behandeln und die erhaltenen Salze auf erhöhte Temperaturen, insbesondere solche oberhalb 100°C, erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

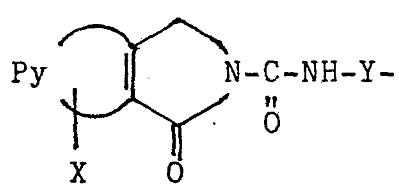

in 4-Stellung substituierten Benzolsulfonamiden umzusetzen. Als solche Ausgangsstoffe kommen beispielsweise infrage

N-Cyclohexyl-harnstoff, die entsprechenden N'-Acetyl, N'-Nitro, N'-Cyclohexyl, N',N'-Diphenyl- (wobei die beiden Phenylreste auch substituiert sowie direkt oder auch über ein Brückenglied wie $-CH_2-$, -NH-, -O- oder -S- miteinander verbunden sein können), N'-Methyl-N'-phenyl, N',N'-Dicyclohexylharnstoffe sowie Cyclohexyl-carbamoyl-imidazole, -pyrazole oder -triazole sowie solche der genannten Verbindungen, die anstelle des Cyclohexyls einen anderen im Bereich der Definition für $R^1$ liegenden Substituenten tragen.

Die Spaltung der als Ausgangsstoffe genannten Benzolsulfonylparabansäuren, -isoharnstoffäther, -isothioharnstoffäther oder -halogenameisensäureamidine erfolgt zweckmäßig durch alkalische Hydrolyse. Isoharnstoffäther können auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagerung von Wasser in die Benzolsulfonylharnstoffe überführt werden.

Die Oxydation von Benzolsulfinyl- bzw. Benzolsulfenylharnstoffen erfolgt nach an sich bekannter Methode, vorzugsweise mit Oxydationsmittels wie Permanganat oder Wasserstoffperoxid.

Die Acylierung der Sulfonylharnstoffe gemäß Verfahren e) kann mit reaktiven Derivaten der Säure

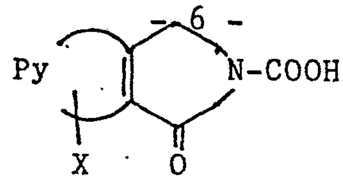

wie beispielsweise Halogeniden oder Urethanen erfolgen. Als Sulfonyl- bzw. Sulfinylhalogenide gemäß Verfahren f) eignen sich insbesondere die Chloride. Als saures Kondensationsmittel kann man beispielsweise Thionylchlorid oder Polyphosphorsäure einsetzen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Synthese der Dihydro-pyrrolo-pyridine erfolgt nach bereits beschriebenen Verfahren (vgl. z.B. Chem. Ber. 82, S. 36, (1949), Chem. Ber. 105, S. 3611, (1972)) oder kann in Analogie dazu durchgeführt werden.

Die Ausführungformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwessenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthesen nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali)-Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignen sich daher als Arzneimittel, insbesondere als Antidiabetika.

Die blutzuckersenkende Wirkung der beschriebenen Benzolsulfonylharnstoffe kann dadurch festgestellt werden, daß man sie als freie Verbindungen oder in Form der Natriumsalze in Dosen von 10 mg oder 2 mg/kg an normal ernährte Kaninchen verfüttert und den Blutzuckerwert nach der bekannten Methode von Hagedorn-Jensen oder mit einem Autoanalyzer über eine längere Zeitdauer ermittelt.

Die Bestimmung der blutzuckersenkenden Wirkung kann aber auch mit geringeren Dosen oder nach anderen bekannten Methoden erfolgen.

Die folgenden Verbindungen I bis III wurden in Dosierungen von 2 mg/kg Kaninchen oral verabreicht und die Blutzuckerwerte wurden mit einem Autoanalyzer über eine läängere Zeitdauer ermittelt. Die hierbei gemessene Blutzuckersenkung ist in der nachfolgenden Tabelle in % nach...Stunden angegeben.

I N-(4-/¯2-(5-Oxo-6,7-dihydro-5H-pyrrolo-/¯3,4-B_7-pyridin-6-yl-carboxamido)-äthyl_7-benzolsulfonyl)-N'-(4-methyl-cyclohexyl)-harnstoff

II N-(4-/¯2-(5-Oxo-6,7-dihydro-5H-pyrrolo-/¯3,4-B_7-pyridin-6-yl-carboxamido)-äthyl_7-benzolsulfonyl)-N'-(4-äthyl-cyclohexyl)-harnstoff

III N-(4-/‾2-(5-Oxo-6,7-dihydro-5H-pyrrolo-/‾3,4-B_7-pyridin-6-yl-carboxamido)-äthyl_7-benzolsulfonyl)-N'-bicyclo/‾2.2.1_7hept-2-yl-methyl-harnstoff

Tabelle

| Verbindung | Blutzuckersenkung am Kaninchen nach oraler Verabreichung von 2 mg/kg in % nach 1 | 3 | 6 | 24 | 48/72 Stunden |
|---|---|---|---|---|---|
| I | | 31 | 34 | 36 | 19/0 |
| II | 36 | 52 | 54 | 48 | 38/0 |
| III | 43 | 26 | 38 | 26 | 0 |

Die erfindungsgemäßen Acylureidoalkylbenzolsulfonylharnstoffe zeichnen sich durch eine starke und langanhaltende blutzuckersenkende Wirkung aus.

Die Eigenschaften der Verbindungen erlauben es, in der Therapie des Diabetes mit so geringen Dosen auszukommen, daß das Präparat nur die verminderte Ansprechbarkeit des Pankreas auf einen erhöhten Blutzuckerspiegel wieder normalisiert.

Benzolsulfonylharnstoffe mit Ureidoalkylrest sind schon mehrfach beschrieben worden (DE-PS 14 43 911, DE-AS 16 70 700, DE-PS 16 18 389, DE-PS 22 38 870). Es war nicht zu erwarten, daß die erfindungsgemäßen Verbindungen sich durch die oben erwähnten günstigen Eigenschaften auszeichnen.

Die erfindungsgemäßen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische

Mittel wie Alkali- oder Erdalkalihydroxyde, -carbonate oder -bicarbonate herangezogen werden. Die Präparate können neben dem Sulfonylharnstoff bzw. dessen Salz auch noch andere Wirkstoffe enthalten.

Als medizinische Präparate kommen vorzugsweise Tabletten in Betracht, die neben den Verfahrenserzeugnissen die üblichen Träger- und Hilfsstoffe wie Talkum, Stärke, Milchzucker oder Magnesiumstearat enthalten. Ein Präparat, das die beschriebenen Benzolsulfonylharnstoffe als Wirkstoff enthält, z.B. eine Tablette oder ein Pulver mit und ohne Zusätze, ist zweckmäßig in eine geeignet dosierte Form gebracht. Als Dosis ist dabei eine solche zu wählen, die der Wirksamkeit des verwendeten Benzolsulfonylharnstoffes und dem gewünschten Effekt angepaßt ist. Zweckmäßig beträgt die Dosierung je Einheit etwa 0,1 bis 10 mg, vorzugsweise 0,5 bis 2 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können. Sie sollen jedoch nicht eine Einschränkung des Erfindungsgegenstandes darstellen.

Beispiel 1:

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexylharnstoff

1,8 g 4-(2-/¯5-Oxo-6,7-dihydro-5H-pyrrolo-/¯3,4-B_7-pyridin-6-yl-carboxamido_7-äthyl)-benzolsulfonamid (Schmp. 250 - 252°C, hergestellt durch Umsetzung von 5-Oxo-6,7-dihydro-5H-pyrrolo-/¯3,4-B_7-pyridin-6-yl-(N-2-phenyl-äthyl)-carboxamid /¯Schmp. 147°C, hergestellt aus 6,7-Dihydro-5H-pyrrolo-/¯3,4-B_7-pyridin-5-on und Phenyläthylisocyanat_7 mit Chlorsulfonsäure und Reaktion des erhaltenen Sulfochlorids mit Ammoniak) werden in 80 ml Aceton nach Zugabe von 1,4 g gemahlener Pottasche mehrere Stunden unter Rückfluß gerührt. Anschließend tropft man 0,7 g Cyclohexylisocyanat, gelöst in wenig Aceton, zu und rührt nochmals unter Rückfluß vier Stunden nach. Nach dem Abkühlen destilliert man im Vakuum das Aceton ab, nimmt den Rückstand in Wasser auf und säuert mit verdünnter Salzsäure auf pH 3-4 an. Der ausgefallene Sulfonylharnstoff wird aus verdünnter Ammoniak-Lösung mit verdünnter Salzsäure umgefällt und aus Äthanol umkristallisiert. Der so erhaltene N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff schmilzt bei 222 - 223°C.

In analoger Weise erhält man den

N-(4-/¯2-(5-Oxo-6,7-dihydro-5H-pyrrolo-/¯3,4-B_7-pyridin-6-yl-carboxamido)-äthyl_7-benzolsulfonyl)-N'-(4-methyl-cyclohexyl)-harnstoff
vom Schmp. 226°C (aus Äthanol)

N-(4-/¯2-(5-Oxo-6,7-dihydro-5H-pyrrolo-/¯3,4-B_7-pyridin-6-yl-carboxamido)-äthyl_7-benzolsulfonyl)-N'-(4-äthyl-cyclohexyl)-harnstoff
vom Schmp. 226°C (aus Äthanol)

N-(4-/¯2-(5-Oxo-6,7-dihydro-5H-pyrrolo-/¯3,4-B_7-pyridin-6-yl-carboxamido)-äthyl_7-benzolsulfonyl)-N'-($\Delta^3$-cyclohexenyl)-harnstoff vom Schmp. 230 - 232°C (aus Äthanol-Dimethylformamid)

N-(4-/¯2-(5-Oxo-6,7-dihydro-5H-pyrrolo-/¯3,4-B_7-pyridin-6-yl-carboxamido)-äthyl_7-benzolsulfonyl)-N'-n-hexyl-harnstoff vom Schmp. 183°C (aus Äthanol)

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclopentyl-harnstoff vom Schmp. 216 - 217°C (aus Äthanol)

N-(4-/¯2-(5-Oxo-6,7-dihydro-5H-pyrrolo-/¯3,4-B_7-pyridin-6-yl-carboxamido)-äthyl_7-benzolsulfonyl)-N'-bicyclo/¯2.2.1_7-hept-2-yl-methyl-harnstoff vom Schmp. 240 - 241°C (aus Äthanol-Dimethylformamid)

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclopentylmethyl-harnstoff vom Schmp. 210 - 211°C (aus Äthanol)

N-(4-/¯2-(5-Oxo-6,7-dihydro-5H-pyrrolo-/¯3,4-B_7-pyridin-6-yl-carboxamido)-äthyl_7-benzolsulfonyl)-N'-(3-methyl-cyclopentylmethyl)-harnstoff vom Schmp. 186 - 188°C (aus Äthanol)

Beispiel 2:

N-(4-/¯2-(1-Oxo-2,3-dihydro-6-methyl-1H-pyrrolo-/¯3,4-C_7-pyridin-2-yl-carboxamido)-äthyl_7-benzolsulfonyl)-N'-cyclohexylharnstoff

1,9 g 4-(2-/¯1-Oxo-2,3-dihydro-6-methyl-1H-pyrrolo-/¯3,4-C_7-pyridin-2-yl-carboxamido_7-äthyl)-benzolsulfonamid (Schmp. 225°C, hergestellt durch Umsetzung von 1-Oxo-2,3-dihydro-6-methyl-1H-pyrrolo-/¯3,4-C_7-pyridin-2-yl-(N-2-phenyl-äthyl)-carboxamid /¯Schmp. 163 - 165°C, hergestellt aus 2-Methyl-5-aminomethyl-isoniotinsäurelactam und Phenyläthylisocyanat_7 mit Chlorsulfonsäure und Reaktion des erhaltenen Sulfochlorids mit Ammoniak) werden in 80 ml Aceton nach Zugabe von 1,4 g gemahlener Pottasche 5 Stunden unter Rühren zum Rückfluß erhitzt. Nach Zugabe von 0,7 g Cyclohexylisocyanat in wenig Aceton wird nochmals 4 Stunden bei Siedetemperatur gerührt. Die erkaltete Suspension wird im Vakuum eingedampft und der Rückstand in Wasser aufgenommen, filtriert und mit verdünnter Salzsäure auf pH 3-4 angesäuert. Der ausgefällte N-(4-/2-(1-Oxo-2,3-dihydro-6-methyl-1H-pyrrolo-/¯3,4-C_7-pyridin-2-yl-carboxamido)-äthyl_7-benzolsulfonyl)-N'-cyclohexyl-harnstoff schmilzt wird nach dem Umfällen aus verdünnter Ammoniak-Lösung mit verdünnter Salzsäure aus Äthanol umkristallisiert. Er schmilzt bei 228°C.

In analoger Weise erhält man den

N-(4-/¯2-(1-Oxo-2,3-dihydro-6-methyl-1H-pyrrolo-/¯3,4-C_7-pyridin-2-yl-carboxamido)-äthyl_7-benzolsulfonyl)-N'-(4-methyl-cyclohexyl)-harnstoff vom Schmp. 206°C (aus Äthanol)

Beispiel 3:

N-(4-/¯2-(3-Oxo-2,3-dihydro-1H-pyrrolo-/¯3,4-C_7-pyridin-2-yl-carboxamido)-äthyl_7-benzolsulfonyl)-N'-cyclohexylharnstoff

0,9 g 4-(2-[3-Oxo-2,3-dihydro-1H-pyrrolo-[3,4-C]-pyridin-2-yl-carboxamido)-äthyl]-benzolsulfonamid (Schmp. 258 - 260°C, hergestellt durch Umsetzung von 3-Oxo-2,3-dihydro-1H-pyrrolo-[3,4-C]-pyridin-2-yl-(N-2-phenyläthyl)-carboxamid [Schmp. 198 - 200°C, hergestellt aus 2,3-Dihydro-1H-pyrrolo-[3,4-C]-pyridin-3-on und 2-Phenyläthylisocyanat] mit Chlorsulfonsäure und Reaktion des erhaltenen Sulfochlorids mit Ammoniak) werden in 50 ml Aceton nach Zusatz von 0,7 g gemahlener Pottasche 6 Stunden unter Rückfluß gekocht. Nach dem Abkühlen fügt man 0,3 g Cyclohexylisocyanat, gelöst in wenig Aceton, zu und rührt weitere 4 Stunden unter Rückfluß nach. Die Suspension wird im Vakuum eingedampft, der Rückstand in Wasser gelöst, filtriert und das Filtrat mit verdünnter Essigsäure angesäuert. der Niederschlag wird abgesaugt, aus verdünnter Ammoniak-Lösung mit verdünnter Essigsäure umgefällt und aus Äthyl-Dimethylformamid umkristallisiert. Der so synthetisierte N-(4-[2-(3-Oxo-2,3-dihydro-1H-pyrrolo-[3,4-C]-pyridin-2-yl-carboxamido)-äthyl]-benzolsulfonyl)-N'-cyclohexyl-harnstoff schmilzt bei 224 - 226°C.

In analoger Weise erhält man den

N-(4-[2-(3-Oxo-2,3-dihydro-1H-pyrrolo-[3,4-C]-pyridin-2-yl-carboxamido)-äthyl]-benzolsulfonyl)-N'-(4-methyl-cyclohexyl)-harnstoff
vom Schmp. 224 - 225°C (aus Äthanol)

N-(4-[2-(3-Oxo-2,3-dihydro-1H-pyrrolo-[3,4-C]-pyridin-2-yl-carboxamido)-äthyl]-benzolsulfonyl)-N'-butylharnstoff
vom Schmp. 209 - 211°C (aus Äthanol)

Beispiel 4:

N-(4-[2-(7-Oxo-5,6-dihydro-7H-pyrrolo-[3,4-B]-pyridin-6-yl-carboxamido)-äthyl]-benzolsulfonyl)-N'-cyclohexyl-harnstoff

1,8 g 4-(2-[7-Oxo-5,6-dihydro-5H-pyrrolo-[3,4-B]-pyridin-6-yl-carboxamido)-äthyl]-benzolsulfonamid (Schmp. 248 - 251°C, hergestellt durch Umsetzung von 7-Oxo-5,6-dihydro-7H-pyrrolo-[3,4-B]-pyridin-2-yl-(N-2-phenyl-äthyl)-carboxamid [Schmp. 172 - 173°C, hergestellt aus 5,6-Dihydro-7H-pyrrolo-[3,4-B]-pyridin-7-on und 2-Phenyläthylisocyanat] mit Chlorsulfonsäure und Reaktion des erhaltenen Sulfochlorids mit Ammoniak) werden zusammen mit 1,4 g gemahlener Pottasche in 75 ml Butanon-2 suspendiert und nach Zugabe von 0,65 g Cyclohexylisocyanat 4 Stunden unter Rückfluß gerührt. Nach dem Abdestillieren des Lösungsmittels wird der Rückstand in Wasser aufgenommen und die Lösung mit verdünnter Essigsäure angesäuert. Der Niederschlag wird abfiltriert und nach dem Umfällen aus verdünnter Essigsäure aus Äthanol umkristallisiert. Der auf diese Weise hergestellte N-(4-[2-(7-Oxo-5,6-dihydro-7H-pyrrolo-[3,4-B]-pyridin-6-yl-carboxamido)-äthyl]-benzolsulfonyl)-N'-cyclohexyl-harnstoff schmilzt bei 220 - 221°C.

In analoger Weise erhält man den

N-(4-[2-(7-Oxo-5,6-dihydro-7H-pyrrolo-[3,4-B]-pyridin-6-yl-carboxamido)-äthyl]-benzolsulfonyl)-N'-butyl-harnstoff
vom Schmp. 219 - 220°C (aus Äthanol)

N-(4-[2-(7-Oxo-5,6-dihydro-7H-pyrrolo-[3,4-B]-pyridin-6-yl-carboxamido)-äthyl]-benzolsulfonyl)-N'-(4-methyl-cyclohexyl)-harnstoff
vom Schmp. 227 - 228°C (aus Äthanol)

N-(4-[2-(7-Oxo-5,6-dihydro-7H-pyrrolo-[3,4-B]-pyridin-6-yl-carboxamido)-äthyl]-benzolsulfonyl)-N'-(3-methyl-cyclopentylmethyl)-harnstoff vom Schmp. 230 - 231°C (aus Äthanol)

Beispiel 5:

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff

1,08 g N-(4-(2-/¯5-Oxo-6,7-dihydro-5H-pyrrolo-/¯3,4-B_7-pyridin-6-yl-carboxamido)-äthyl_7-benzolsulfonyl)-N'-cyclohexyl-thioharnstoff (Schmp. 208 - 210°C, hergestellt aus dem entsprechenden Sulfonamid und Cyclohexylsenföl) werden in 75 ml Wasser und 75 ml Methanol nach Zugabe von 0,64 g gelbem Quecksilberoxid 8 Stunden unter Rückfluß gerührt. Nach dem Abkühlen filtriert man das Quecksilbersulfid ab, dampft das Filtrat im Vakuum ein und kristallisiert den Rückstand aus Äthanol um. Der so hergestellte N-(4-/2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff schmilzt bei 221 - 223°C.

Beispiel 6:

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff

1,08 g N-(4-(2-/¯5-Oxo-6,7-dihydro-5H-pyrrolo-/¯3,4-B_7-pyridin-6-yl-carboxamido)-äthyl_7-benzolsulfonyl)-N'-cyclohexyl-thioharnstoff (Schmp. 208 - 210°C, hergestellt aus dem entsprechenden Sulfonamid und Cyclohexylsenföl) werden in 75 ml wasserfreiem Methanol nach Zusatz von 0,64 g gelbem Quecksilberoxid mehrere Stunden unter Rückfluß gerührt. Nach dem Erkalten wird das Quecksilbersulfid abfiltriert und das Filtrat eingedampft.
Der zurückgebliebene N-(4-/2-(5-Oxo-6,7-dihydro-5H-pyrrolo-/¯3,4-B_7-pyridin-6-yl-carboxamido)-äthyl_7-benzolsulfonyl)-N'-cyclohexyl-isoharnstoff-methyläther wird als Rohprodukt in 30 ml conc. Salzsäure zwei Stunden bei 50°C gerührt. Nach dem Verdünnen mit 30 ml Wasser wird auf pH 3 - 4 gestellt.

Der auskristallisierte N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff wird abgesaugt und aus Äthanol umkristallisiert. Er schmilzt nach dem Trocknen bei 222 - 223°C.

Beispiel 7:

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff

1,52 g (0,01 Mol) Cyclohexylharnstoff werden in 50 ml Tetrahydrofuran nach Zusatz von 0,6 g Natriumhydrid-Dispersion (55 %-ig) 3 Stunden unter Rückfluß gerührt. Nach dem Abkühlen tropft man eine Lösung von 3,8 g 4-/¯2-(5-Oxo-6,7-dihydro-5H-pyrrolo-/¯3,4-B_7-pyridin-6-yl-carboxamido)-äthyl_7-benzolsulfonsäurechlorid (hergestellt wie in Beispiel 1 beschrieben) in 20 ml Tetrahydrofuran zu und kocht weitere 3 Stunden. Nach dem Erkalten wird der Niederschlag abgesaugt, in Wasser aufgenommen und auf pH 3 - 4 angesäuert. Anschließendes Umfällen aus verdünnter Ammoniaklösung mit verdünnter Salzssäure und Umkristallisieren aus Äthanol liefert den N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff vom Schmp. 222 - 223°C.

Beispiel 8:

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff

2,01 g 6,7-Dihydro-5H-pyrrolo-/¯3,4B_7pyridin-5-on werden in 50 ml absolutem Toluol mit 0,72 g Natriumhydrid-Dispersion (55 %-ig in Paraffin) versetzt und 6 Stunden unter Rückfluß gerührt. Nach dem Abkühlen setzt man 15 ml einer 25 %-igen Phosgen-Lösung in Toluol zu und rührt 3 Stunden bei 50°C nach. Die so erhaltene Lösung des Carbamoylchlorids wird filtriert und bei Raumtemperatur zu einer Suspension von 3,16 g N-(4-/¯2-Aminoäthyl_7-

benzolsulfonyl)-N'-cyclohexyl-harnstoff und 2,8 ml Triäthylamin in 50 ml Tetrahydrofuran getropft. Man rührt 3 Stunden bei Raumtemperatur nach und hält den pH-Wert der Mischung im alkalischen Bereich. Anschließend engt man im Vakuum ein, fällt den Rückstand aus verdünnter Ammoniaklösung mit verdünnter Essigsäure um und kristallisiert aus Äthanol um. Der so dargestellte N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff zeigt im DC-Vergleich und im Mischschmelzpunkt keinen Unterschied zu einer nach Beispiel 1 hergestellten Probe.

Beispiel 9:

N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff

2,01 g N-(4-/¯2-(5-Oxo-6,7-dihydro-5H-pyrrolo-/¯3,4-B_7-pyridin-6-yl-carboxamido)-äthyl_7-benzolsulfonyl)-harnstoff (vom Schmp. 243 - 245°C, hergestellt aus dem entsprechenden Benzolsulfonamid und Kaliumcyanat) werden in 50 ml Dioxan nach dem Zusatz von 1,03 g Cyclohexylamin 3 Stunden unter Rühren zum Rückfluß erhitzt. Beim Abkühlen fällt ein farbloser Niederschlag aus. Man saugt ab, suspendiert in 50 ml Wasser und säuert mit verdünnter Essigsäure an. Der so erhaltene N-(4-/¯2-(5-Oxo-6,7-dihydro-5H-pyrrolo-/¯3,4-B_7-pyridin-6-yl-carboxamido)-äthyl_7-benzolsulfonyl)-N'-cyclohexyl-harnstoff wird abgesaugt und aus Äthanol-Dimethylformamid umkristallisiert. Der Schmelzpunkt liegt bei 220 - 222°C. Ein DC-Vergleich und ein Mischschmelzpunkt mit einer nach Beispiel 1 hergestellten Probe zeigen Übereinstimmung.

In analoger Weise erhält man den

N-(4-/¯2-(5-Oxo-6,7-dihydro-5H-pyrrolo-/¯3,4-B_7-pyridin-6-yl-carboxamido)-äthyl_7-benzolsulfonyl)-N'-(4-chlor-cyclohexyl)-harnstoff
vom Schmp. 220 - 221°C (aus Äthanol-Dimethylformamid)

N-(4-/¯2-(5-Oxo-6,7-dihydro-5H-pyrrolo-/¯3,4-B_7-pyridin-6-yl-carboxamido)-äthyl_7-benzolsulfonyl)-N'-(4-methoxy-cyclohexyl)-harnstoff vom Schmp. 190 - 192°C (aus Essigester)

Beispiel 10

N-(4-/¯2-(5-Oxo-2,3-dihydro-5H-pyrrolo-/¯3,4-B_7-pyridin-6-yl-carboxamido)-äthyl_7-benzolsulfonyl)-N'-cyclohexyl-harnstoff

6,9 g (0,02 Mol) 4-(2-/¯5-Oxo-2,3-dihydro-5H-pyrrolo-/¯3,4-B_7-pyridin-6-yl-carboxamido_7-äthyl)-benzolsulfinsäure, (Fp: 253 - 255°C Zers., hergestellt durch Umsetzung von 5-Oxo-2,3-dihydro-5H-pyrrolo-/¯3,4-B_7-pyridin-2-yl-(N-2-phenyläthyl)-carboxamid /¯Schmp. 147 - 148°C, hergestellt aus 2,3-Dihydro-5H-pyrrolo-/¯3,4-B_7-pyridin-5-on und 2-Phenyläthyl-isocyanat_7 mit Chlorsulfonsäure und Reaktion des erhaltenen Sulfochlorids mit Natriumsulfit in Chloroform und Wasser) werden mit 7,2 ml Thionylchlorid übergossen. Das abgesaugte Sulfinylchlorid wird mit Cyclohexyl-harnstoff in Pyridin zum N-(4-/¯2-(5-Oxo-2,3-dihydro-5H-pyrrolo-/¯3,4-B_7-pyridin-6-yl-carboxamido)-äthyl_7-benzolsulfinyl)-N'-cyclohexyl-harnstoff umgesetzt. Durch Oxidation mit Permanganat in DMF erhält man den N-(4-/¯2-(5-Oxo-2,3-dihydro-5H-pyrrolo-/¯3,4-B_7-pyridin-6-yl-carboxamido)-äthyl_7-benzolsulfonyl)-N'-cyclohexyl-harnstoff vom Schmp. 221 - 223°C.

PATENTANSPRÜCHE:

1. Sulfonylharnstoffe der Formel

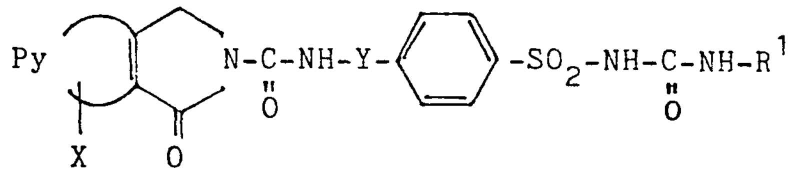

in welcher bedeuten

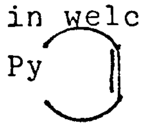 einen Pyridin-Ring, der das Stickstoffatom an den vier möglichen Positionen tragen kann

X Wasserstoff, Alkyl mit 1 - 4 C-Atomen, Halogen

Y Alkylen mit 2 - 3 C-Atomen

$R^1$ Alkyl von 4 - 8 C-Atomen, Cycloalkyl, Alkylcycloalkyl, Dialkylcycloalkyl, Cycloalkylalkyl, Cycloalkenyl, Alkylcycloalkenyl mit jeweils 5 - 9 C-Atomen, Methylcyclopentylmethyl, Cyclohexenylmethyl, Chlorcyclohexyl, Methoxycyclohexyl, Bicycloheptyl, Bicycloheptenyl, Bicyclohetylmethyl, Bicycloheptenylmethyl, Bicyclooctyl, Nortricyclyl, Adamantyl oder Benzyl,

und deren physiologisch verträglichen Salze.

2. Verfahren zur Herstellung von Sulfonylharnstoffen gemäß Anspruch 1, dadurch gekennzeichnet, daß man

a) mit der Gruppe

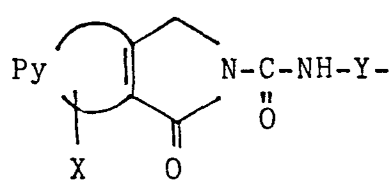

in 4-Stellung substituierte Benzolsulfonyl-isocyanate, -carbaminsäureester, - thiolcarbaminsäureester, -harnstoffe, -semicarbazide oder -semicarbazone mit einem Amin $R^1-NH_2$ oder dessen Salzen umsetzt oder Sulfonamide der Formel

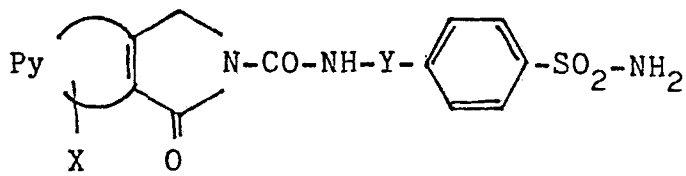

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte Benzolsulfonyl-isoharnstoffäther, -isothioharnstoffäther, -parabansäuren oder -halogenameisensäureamidine spaltet,

c) in

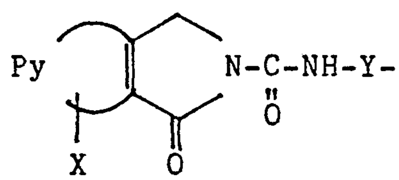

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffen der Formel

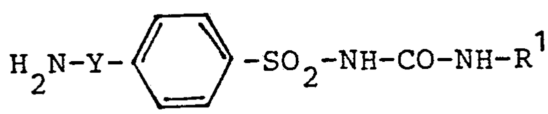

gegebenenfalls stufenweise den Rest

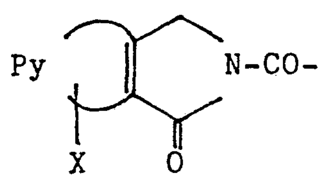

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^1$-N'-hydroxy-harnstoff umsetzt
und die Reaktionsprodukte gegebenenfalls zur Salzbildung mit alkalischen Mitteln behandelt.

3. Arzneimittel auf Basis eines Sulfonylharnstoffes gemäß Anspruch 1 oder eines seiner Salze.

4. Verwendung eines Sulfonylharnstoffes gemäß Anspruch 1 oder eines seiner Salze bei der Bekämpfung von Diabetes.

5. Verfahren zur Herstellung eines Arzneimittels gemäß Anspruch 3, dadurch gekennzeichnet, daß man einen Sulfonylharnstoff der im Anspruch 1 angegebenen Formel oder eines seiner Salze in eine geeignete Applikationsform bringt.

6. Verfahren zur Senkung des Blutzuckerspiegels bei der Behandlung der Diabetes, dadurch gekennzeichnet, daß man eine wirksame Menge einer Verbindung gemäß Anspruch 1 verabreicht.

PATENTANSPRUCH ÖSTERREICH:

1. Verfahren zur Herstellung von Sulfonylharnstoffen der Formel

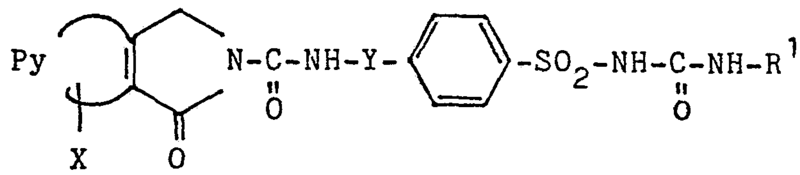

in welcher bedeuten

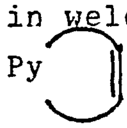 einen Pyridin-Ring, der das Stickstoffatom an den vier möglichen Positionen tragen kann

X Wasserstoff, Alkyl mit 1 - 4 C-Atomen, Halogen

Y Alkylen mit 2 - 3 C-Atomen

$R^1$ Alkyl von 4 - 8 C-Atomen, Cycloalkyl, Alkylcycloalkyl, Dialkylcycloalkyl, Cycloalkylalkyl, Cycloalkenyl, Alkylcycloalkenyl mit jeweils 5 - 9 C-Atomen, Methylcyclopentylmethyl, Cyclohexenylmethyl, Chlorcyclohexyl, Methoxycyclohexyl, Bicycloheptyl, Bicycloheptenyl, Bicyclohetylmethyl, Bicycloheptenylmethyl, Bicyclooctyl, Nortricyclyl, Adamantyl oder Benzyl,

und von deren physiologisch verträglichen Salzen, dadurch gekennzeichnet, daß man

a) mit der Gruppe

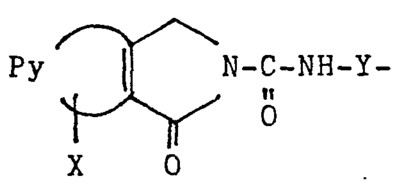

in 4-Stellung substituierte Benzolsulfonyl-isocyanate, -carbaminsäureester, - thiolcarbaminsäureester, -harnstoffe, -semicarbazide oder -semicarbazone mit einem Amin $R^1$-$NH_2$ oder dessen Salzen umsetzt oder Sulfonamide der Formel

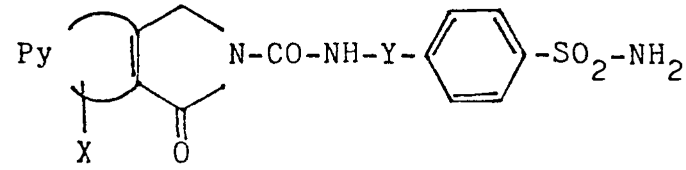

oder deren Salze mit $R^1$-substituierten Isocyanaten, Carbaminsäureestern, Thiolcarbaminsäureestern, Carbaminsäurehalogeniden oder Harnstoffen umsetzt,

b) entsprechend substituierte Benzolsulfonyl-isoharnstoffäther, -isothioharnstoffäther, -parabansäuren oder -halogenameisensäureamidine spaltet,

c) in

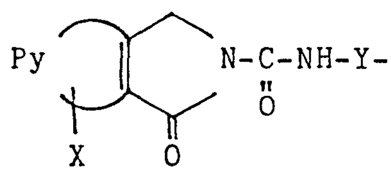

substituierten Benzolsulfonylthioharnstoffen das Schwefelatom durch Sauerstoff ersetzt,

d) entsprechende Benzolsulfinyl- oder -sulfenyl-harnstoffe oxydiert,

e) in Benzolsulfonylharnstoffen der Formel

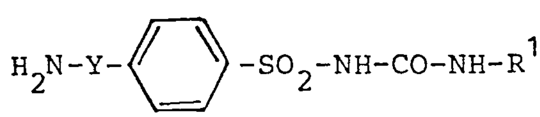

gegebenenfalls stufenweise den Rest

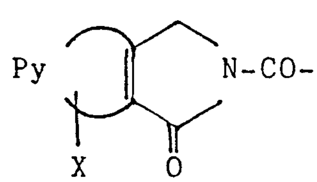

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^1$-N'-hydroxy-harnstoff umsetzt

und die Reaktionsprodukte gegebenenfalls zur Salzbildung mit alkalischen Mitteln behandelt.

0029983

Nummer der Anmeldung

EP 80107307.3

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int Cl ³) |
|---|---|---|---|
| | DE - A1 - 2 621 958 (HOECHST)<br>+ Ansprüche; Seite 10, 3. Absatz - Seite 14, erster Absatz +<br>---- | 1-3,5 | C 07 D 471/04<br>A 61 K 31/64//<br>(C 07 D 471/04<br>C 07 D 221/00<br>C 07 D 209/00) |

RECHERCHIERTE SACHGEBIETE (Int. Cl.³)

C 07 D 213/00
C 07 D 471/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5

Unvollständig recherchierte Patentansprüche: -

Nicht recherchierte Patentansprüche: 4,6

Grund für die Beschränkung der Recherche:

Verfahren zur therapeutischen Behandlung des menschlichen und tierischen Körpers (Art. 52(4) EPÜ)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 11-02-1981 | TENGLER |

EPA Form 1505.1 06.78